# EUROPEAN PATENT SPECIFICATION

(11) **EP 3 673 984 B1**
(45) Date of publication and mention of the grant of the patent: **21.07.2021**
(21) Application number: 18216011.9
(22) Date of filing: 31.12.2018
(51) Int. Cl.: B01J 13/14, C11D 3/50

(54) **PERFUME MICROENCAPSULATION**
PARFÜMMIKROVERKAPSELUNG
MICROENCAPSULATION DE PARFUM

(43) Date of publication of application: 01.07.2020
(73) Proprietor: Clariant International Ltd, 4132 Muttenz (CH)
(72) Inventor: WAGDARE, Nagesh Appasaheb, 400708 Airoli, Navi Mumbai (IN); PANDEY, Someshwarnath Dinanath, 400708 Airoli, Navi Mumbai (IN)
(74) Representative: Kampen, Daniela

(56) References cited:
- EP-A2- 3 300 794
- WO-A1-2016/090623
- WO-A1-2016/100477
- WO-A1-2017/091421
- WO-A1-2018/115330
- WO-A1-2018/172514
- US-A1- 2015 098 979

## Description

The present invention relates to perfume-containing microcapsules, aqueous dispersions of the perfume-containing microcapsules and methods of forming perfume-containing microcapsules. The invention is also concerned with personal care or fabric care products containing said microcapsules.

Methods and technologies for delaying the release of perfumes, including microencapsulation, are known in the art - reference may be made to WO 01/93813 A2.

Microcapsules may be so-called "core-shell" microcapsules, which consist of a generally spherical shell that is formed around a core containing the perfume and/or other materials, which it is desired should be encapsulated. The shell may have a barrier function thereby protecting the contents from the environment external of the microcapsule. Reference may be made to *"*Synthesis of Core-Shell Polyurethane-Polydimethylsiloxane Particles by Polyaddition in Organic Dispersant Media: Mechanism of Particle Formation", Chambon et al, Macromol. Symp., 2005, 227-238.

Perfumes may rapidly evaporate from the surface they are intended to bestow a fragrance to, all the more if the fragrance oil is a volatile "top note". If the surface is warm, such as human skin, then the fragrance oil may evaporate very quickly. Microencapsulation may provide a way to ensure perfume longevity. In the case of perfumes, therefore, the shell may also act as a means of controlling release of perfume.

The nature and composition of the shell may influence the manner in which perfume is released from a core-shell microcapsule. A shell may be water soluble or water swellable or perfume-release may be triggered by exposure of the capsules to a moist environment. Alternatively, if a shell is temperature-sensitive, a microcapsule may release perfume in response to elevated temperatures. Microcapsules may instead release perfume in response to shear forces applied to the surface of the microcapsules.

A variety of methods is known for the production of core-shell capsules. One such method is interfacial polymerisation. Interfacial polymerisation, typically proceeds with the formation of a fine dispersion or emulsion of oil droplets (the oil droplets will contain perfume or any other material that is to be encapsulated) in an aqueous continuous phase. The dispersed droplets form the core of the future capsule and the dimensions of the dispersed droplets determine the size of the subsequent capsules.

The emulsion is obtained through vigorous stirring of oil phase and the aqueous continuous phase, typically in the presence of an emulsifier. The interfacial polymerization process comprises two steps: during the first step, the diffusion of two different reactive monomers typically occurs, and they come in contact at the interface, i.e., at the surface of the oil droplets of the emulsion system, leading to the formation of an initial thin polymeric layer shell. During a second step, an increase of the shell thickness occurs, by the continuation of the polymerization reactions towards the organic (dispersed) phase. The polymerization is controlled by diffusion, so the growth speed of the microcapsule's shell will decrease as the shell thickness increases. By means of the appropriate selection of wall-forming materials, cross-links may be formed as the polymer wall forms. The extent of cross-linking may affect such factors as the hardness, brittleness, and permeability of the capsule wall. Interfacial polymerisation may offer formulators a convenient and versatile means for encapsulating perfumes as well as other ingredients.

WO 2018/172514 A1 discloses a microcapsule comprising a cross-linked polymeric shell encapsulating a perfume oil core, wherein the cross-linked polymeric shell comprises a mixture of polysiloxane and a melamine formaldehyde resin cross-linked with polyisocyanate.

It may be challenging, when using microencapsulated perfumes, to attach them to the substrate-to-be-perfumed. This may be a particular problem if the substrate is hair or a fabric which is to be washed with a product comprising microencapsulated perfume. Such a product will also typically comprise components, such as surfactants, intended to remove whatever is attaching to the substrate surface, so that it is likely that microencapsulated perfume will be washed off.

It is against this background that the invention has been devised.

According to a first aspect of the invention, a core-shell microcapsule is provided comprising a cross-linked polymeric shell encapsulating a perfume-containing core, wherein the cross-linked polymeric shell is the reaction product of a functionalized polysiloxane, a polyisocyanate and a polyamine and wherein the functionalized polysiloxane comprises a polysiloxane backbone, one or more ethoxylated alcohol side chains and one or more substituted or unsubstituted alkyl or aliphatic cycloalkyl side chains.

As used herein, the term "microcapsule" refers to capsules which have a median particle size d(50) from 0.1 to 1000 micrometers, preferably from 0.1 to less 100 micrometers, more preferably from 1 to 30 micrometers.

The median particle size d(50) is determined by laser diffraction analysis, for example by using a Horiba LA 940 or Mastersizer 3000 from Malvern using the "Mie Scattering Theory" evaluation. Median values are defined as the value where half of the population resides above this point, and half resides below this point. For particle size distributions the median is called the "d(50)" or "D50".

As used herein, the term "polyisocyanate" refers to a molecule comprising two or more isocyanate functional groups.

As used herein, the term "polyamine" refers to a molecule comprising two or more amine functional groups.

All percentages are by weight (w/w) of the total composition. All ratios are weight ratios. "wt.%" means percentage by weight. References to 'parts' e.g. a mixture of 1 part X and 3 parts Y, is a ratio by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams" and "kg" means "kilogram" or "kilograms". Herein, "comprising" means that other steps and other ingredients can be in addition. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. "Molecular weight" or "M.Wt." or "MW" and grammatical equivalents mean the number average molecular weight.

The shell formation is due to interfacial polymerization between -NCO groups from the polyisocyanate and the hydroxyl groups present in the functionalized polysiloxane. This gives rise to silicone-urethane linkages. Unreacted -NCO groups react further with amines in the polyamine. The reaction between -NCO groups and amino groups leads to the formation of urea linkages.

The core-shell microcapsule according to the invention protects the encapsulated perfume from dissipation in an aqueous and/or elevated temperature environment and the microcapsules are not ruptured by the levels of shear encountered in hair drying with a towel after washing with shampoo. The core-shell microcapsules according to the invention facilitate controlled perfume-release by means of diffusion through the shell wall of the microcapsule.

The functionalized polysiloxane present in the core-shell microcapsules according to the invention comprises both hydrophilic and hydrophobic segments. The substituted or unsubstituted alkyl or aliphatic cycloalkyl side chain(s) and the polysiloxane backbone are hydrophobic and have an affinity for the surface of the hair which also tends to be hydrophobic. Furthermore, the cross-linked polymer of the shell tends to be flexible, elastic and "sticky", which may also assist in causing it to adhere to hair. These aspects promote adherence of the core-shell microcapsules to the hair and resist removal during washing processes.

The ethoxylated side chain(s) are hydrophilic and tend to orient themselves outwards from the shell toward the aqueous phase enabling emulsification. Advantageously, the functionalized polysiloxane comprises 10 or more, preferably 15 or more ethoxylated alcohol side chains.

Advantageously, the functionalized polysiloxane has Structure I: 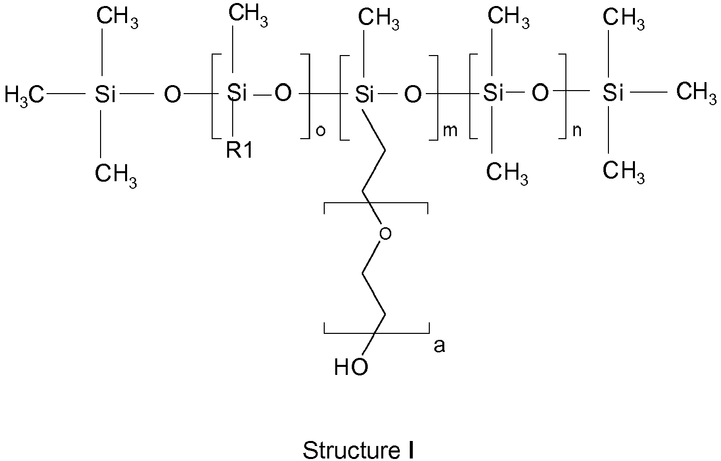 where:
R1 is a substituted or unsubstituted alkyl or is a substituted or unsubstituted aliphatic cycloalkyl group having from 1 to 20 carbon atoms;
a is from 1 to 25, preferably 5 to 15;
m+n+o is 100; m+o is from 10 to 30; and n is from 70 to 90.

More advantageously, in Structure I:
R1 is a straight chain C16 alkyl group;
a is 10;
n is 75;
m+o is 25. Preferably, o is from 2 to 5 and m is from 20 to 23.

The molecule according to Structure I may be synthesized from a trimethylsiloxane-terminated copolymer of methyl hydrosiloxane and dimethylsiloxane, by reaction of the copolymer with an allyl alcohol ethoxylate and an alkene in the presence of a catalyst, such as a platinum catalyst.

The polyisocyanate may be selected from an aromatic polyisocyanate, an aliphatic polyisocyanate and mixtures thereof. Advantageously, the polyisocyanate is selected from isophorone diisocyante, toluene diisocyante, hexamethylene diisocyanate, toluene triisocyante, 1,4-phenylene diisocyanate, 1,3-phenylene diisocyanate, m-xylylene diisocyanate, tolylene-2,4-diisocyanate, tolylene-2,6-diisocyanate, poly(hexamethylene diisocyanate), trans-1,4-cyclohexylene diisocyanate, polypropylene glycol) terminated with tolylene 2,4-diisocyanate, tolylene diisocyanate, 1,4-diisocyanatobutane, 2,4-toluene diisocyanate, 2,6-toluene diisocyanate, 1,8-diisocyanatooctane, 3,3'-dimethyl-4,4'-biphenylene diisocyanate, 4,4'-Methylenebis(phenyl isocyanate) and mixtures thereof. More advantageously still, the polyisocyanate comprises and preferably consists of isophorone diisocyanate.

The polyamine may be selected from diethylenetriamine, bis(hexamethylene)triamine, triamine tetraacetate, melamine-(triamine-15N3), bis(hexamethylene)triamine, (3-trimethoxy silyl propyl) diethylenetriamine, N-cyclopropyl-2,4,6-triamino-1,3,5-triazine, melamine-13C3, N(2),N(4),N(6)-tris(2-phenylethyl)-1,3,5-triazine-2,4,6-triamine, N(2),N(4),N(6)-tris(4-chlorophenyl)-1,3,5-triazine-2,4,6-triamine, N,N'-di-tert-butyl-N", N,N-dimethyl-1,3,5-triazine-2,4,6-triamine and mixtures thereof. Advantageously, the polyamine comprises and preferably consists of diethylenetriamine.

Suitable perfume oils which may be encapsulated according to the invention include anethole, benzaldehyde, decyl aldehyde, amyl acetate, benzyl acetate, benzyl alcohol, benzyl formate, benzyl propionate, iso-bornyl acetate, camphene, cis-citral (neral), citronellal, citronellol, citronellyl acetate, paracymene, decanal, dihydrolinalool, dihydromyrcenol, dimethyl phenyl carbinol, eucalyptol, geranial, geraniol, geranyl acetate, geranyl nitrile, helional, cis-3-hexenol, cis-3-hexenyl acetate, dipropylene glycol, diethyl phthalate, phenyl ethyl acetate, dihydrocitronellal, d-limonene, linalool, linalool oxide, tetra-hydro linalool, linalyl acetate, linalyl propionate, methyl anthranilate, alpha-methyl ionone, methyl nonyl acetaldehyde, methyl phenyl carbinyl acetate, laevo-menthyl acetate, menthone, iso-menthone, myrcene, myrcenyl acetate, myrcenol, nerol, neryl acetate, nonyl acetate, prenyl acetate, manjantol, ambrettolide, ambroxan, cetelox, phenyl ethyl alcohol, phenyl acetaldehyde, alpha-pinene, beta-pinene, gamma-terpinene, alpha-terpineol, beta-terpineol, terpinyl acetate, vertenex (para-tertiary-butyl cyclohexyl acetate), alpha damascone, damascone beta, undecalactone, undecylenic aldehyde, amyl cinnamic aldehyde, iso-amyl salicylate, beta-caryophyllene, cedrene, cinnamic alcohol, coumarin, cymal, dimethyl benzyl carbinyl acetate, dimethyl benzyl carbinol, ethyl vanillin, eugenol, iso-eugenol, dihydro-norcyclopentadienyl acetate, dihydro-nor-cyclopentadienyl propionate, heliotropine, cyclohexyl salicylate, 3-cis-hexenyl salicylate, hexyl salicylate, lilial (para-tertiarybutyl-alpha-methyl hydrocinnamic aldehyde), gamma-methyl ionone, nerolidol, patchouli alcohol, phenyl hexanol, beta-selinene, trichloromethyl phenyl carbinyl acetate, triethyl citrate, vanillin, veratraldehyde, 2-methyl-3-(para tert butylphenyl)-propionaldehyde, benzophenone, benzyl salicylate, ethylene brassylate, galaxolide (1,3,4,6,7,8-Hexahydro-4,6,6,7,8,8-hexamethyl-cyclopenta-gamma-2-benzopyra n), hexyl cinnamic aldehyde, lyral (4-(4-hydroxy-4-methylpentyl)-3-cyclohexene- 10-carboxaldehyde), methyl cedrylone, dihydro isojasmonate, methyl dihydro jasmonate, methyl-beta-naphthyl ketone, gamma decalactone, musk indanone, musk ketone, musk tibetene, phenylethyl phenyl acetate, cyfleural S6MC, PE isobutyrate, PE propionate, tripal, ligustral, and mixtures thereof.

According to a second aspect of the invention, an aqueous dispersion or emulsion is provided comprising core-shell microcapsules according to the first aspect of the invention.

According to a third aspect of the invention, personal care or fabric care products are provided, comprising core-shell microcapsules according to the first aspect of the invention.

A personal care product according to the invention may additionally comprise one or more ingredients common in the field of cosmetology, pharmacy, and dermatology. Such additional ingredients may include oils, petrolatum, fatty alcohols, silicones, waxes, emulsifiers, co-emulsifiers, cationic polymers, film-formers, superfatting agents, stabilizers, polyols, preservatives, pearlizing agents, opacifiers, dyes, fragrances, solvents, protein derivatives such as gelatin, collagen hydrolysates, natural or synthetic-based polypeptides, egg yolk, lecithin, lanolin and lanolin derivatives and mixtures thereof.

A fabric care product according to the invention may additionally comprise one or more ingredients common in the field of laundry and fabric care. Such additional ingredients may include surfactants, emulsifiers, builders, bleaching catalysts and bleach activators, sequestrants, graying inhibitors, color transfer inhibitors, color fixatives, enzymes, optical brighteners, softening components, dyes, perfumes and mixtures thereof.

According to a fourth aspect of the invention, a method is provided of making an aqueous dispersion of core-shell microcapsules comprising a cross-linked polymeric shell encapsulating a perfume-containing core, the method comprising:
a. Forming a disperse phase comprising a perfume and a polyisocyanate;
b. Forming an aqueous continuous phase;
c. Combining the disperse phase with the aqueous continuous phase and mixing to form a emulsion of the dispersed phase in the aqueous continuous phase;
d. Adding a functionalized polysiloxane to the emulsion and mixing, wherein the functionalized polysiloxane comprises a polysiloxane backbone, at least one ethoxylated alcohol side chain and one or more substituted or unsubstituted alkyl or aliphatic cycloalkyl side chains;
e. Adding a polyamine to the mixture and mixing to form core-shell microcapsules dispersed in the aqueous continuous phase.

Advantageously, according to the method of the fourth aspect of the invention, mixing in d. is performed for 1-4 hours. Mixing in e. is also advantageously performed for 1-5 hours.

Preferably, according to the method of the fourth aspect of the invention, the functionalized polysiloxane has Structure I: 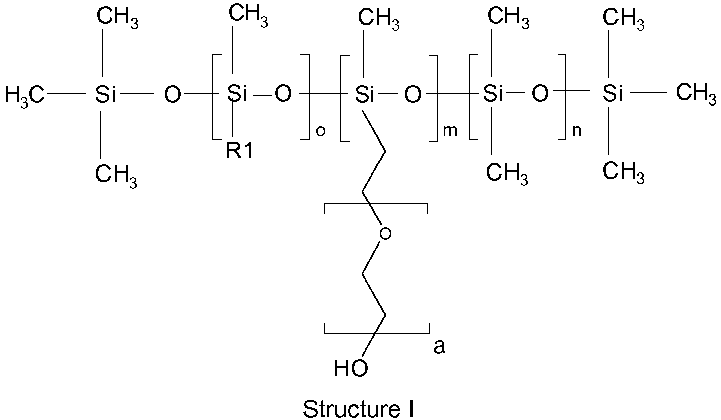 where:
R1 is a substituted or unsubstituted alkyl or is a substituted or unsubstituted aliphatic cycloalkyl group having from 1 to 20 carbon atoms;
a is from 1 to 25, preferably 5 to 15;
m+n+o is 100; m+o is from 10 to 30; and n is from 70 to 90.

Preferably, in Structure I:
R1 is a straight chain C16 alkyl group;
a is 10;
n is 75;
m+o is 25. Preferably, o is from 2 to 5 and m is from 20 to 23.

According to the method of the fourth aspect of the invention, the polyisocyanate may be selected from any of the polyisocyanates listed in relation to the first aspect of the invention. Advantageously, the polyisocyanate comprises and preferably consists of isophorone diisocyanate.

According to the method of the fourth aspect of the invention, the polyisamine may be selected from any of the polyamines listed in relation to the first aspect of the invention. Advantageously, the polyamine comprises and preferably consists of diethylenetriamine.

An indicative reaction to make the core-shell microcapsules according to the invention may proceed in the following way, in which the reaction product may be as shown. The polyisocyanate is isophorone diisocyanate and the polyamine is diethylenetriamine (DETA). The dotted line on the left hand side of the reaction product indicates that the polymer continues beyond what it shown: 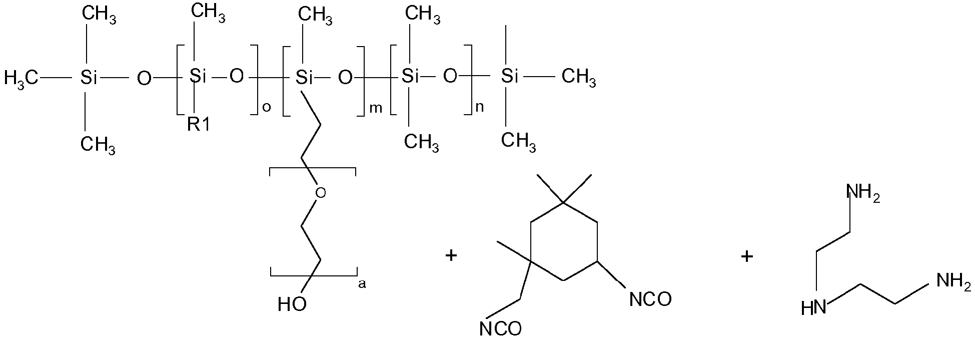 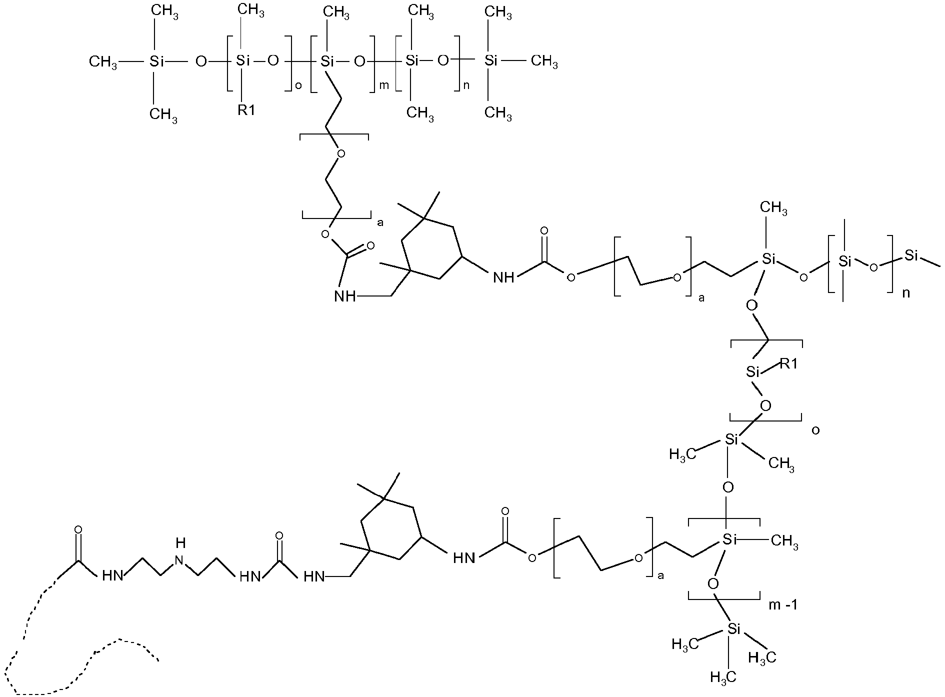 where:
R1 is a substituted or unsubstituted alkyl or is a substituted or unsubstituted aliphatic cycloalkyl group having from 1 to 20 carbon atoms;
a is from 1 to 25, preferably 5 to 15;
m+n+o is 100; m+o is from 10 to 30; and n is from 70 to 90.

Preferably, in Structure I:
R1 is a straight chain C16 alkyl group;
a is 10;
n is 75;
m+o is 25. Preferably, o is from 2 to 5 and m is from 20 to 23.

According to the method of the fourth aspect of the invention, one or more additional materials may be present in the aqueous phase, such as a catalyst for the polymerization, a rheology modifier, a stabilizing agent or mixtures thereof.

In one embodiment, the following additional materials may be added to the aqueous phase:

| **Name or Abbreviation** | **Chemical Name** | **Function** |
|---|---|---|
| PVA | Polyvinyl alcohol | Emulsifier |
| DABCO | 1,4-diazabicyclo[2.2.2]octane | Catalyst for polymerization |
| Aristoflex TAC (Clariant) | Ammonium Acryloyl Dimethyllaurate/Carboxyethyl Acrylate Crosspolymer | Rheology modifier and stabilizer |

### LABORATORY SYNTHESIS OF CORE-SHELL MICROCAPSULES

An aqueous dispersion of core-shell microcapsules according to the invention may be synthesized as follows:

| | **Materials** | **Mass (g)** | **%wt** |
|---|---|---|---|
| **A: Disperse Phase** | Perfume oil | 17.5 | 35 |
| | Isophorone Diisocyanate | 0.222 | 0.444 |
| **B: Aqueous Contiuous Phase** | 3% PVA + 0.5% Aristoflex TAC aqueous solution | 29.47 | 60.91 |
| | DABCO | 0.009 | 0.018 |
| **C** | Functionalized polysiloxane¹ | 1.79 | 3.58 |
| **D** | Diethylenetriamine (DETA) (0.011% aqueous solution) | 0.022 | 0.044 |
| **Total** | | **50** | **100** |

| | | | |
|---|---|---|---|
| ¹ The functionalized siloxane corresponded to Structure I, in which: R1 is a C16 alkyl group a is 10 n is 75; o is 2 m is 23 | | | |

### Method

### Step 1: Preparation of Emulsion

1. Weigh Part A (disperse phase) into 100ml glass beaker.
2. Weigh Part B (aqueous continuous phase) materials into 100ml glass beaker.
3. Assemble the beaker containing Part B (aqueous continuous phase) under IKA Eurostar 60 laboratory stirrer.
4. Now add Part A (disperse phase) from beaker to Part B (aqueous continuous phase) slowly over a period of 7 minutes at 1500rpm to ensure proper mixing of two parts. After complete addition continue stirring for 15 minutes.

### Step 2: Formation of Capsules

1. After 15min emulsion add Part C (Functionalized Polysiloxane) and continue stirring at 1200rpm for next two hours.
2. After two hours add Part-D (DETA Solution) and continue stirring at 900rpm for next 3 hours.
3. After three hours stop the reaction, collect the sample.

### EFFICACY TEST ON HUMAN HAIR

Hair wash and fragrance evaluation procedure:
1. Apply 2g of hair shampoo on wet hair tress. The hair shampoo formulation is given below (see "Hair Shampoo Formulation" under "Product Examples") and comprises core-shell microcapsules made according to the laboratory synthesis, above.
2. Rub hair tress for 3 minutes to spread the shampoo evenly.
3. Rinse hair tress under running tap water for 1minute.
4. Use fingers to remove excess water from hair tress and further dab dry with tissue towel.
5. Dry hair tress in humidity (60-63% Relative humidity) and temperature (room temperature) controlled environment for 12 hours.
6. Panelist to comb dry hair tress (coded) 3 times and conduct smell evaluation.

The results of the evaluation are presented in Table 1.

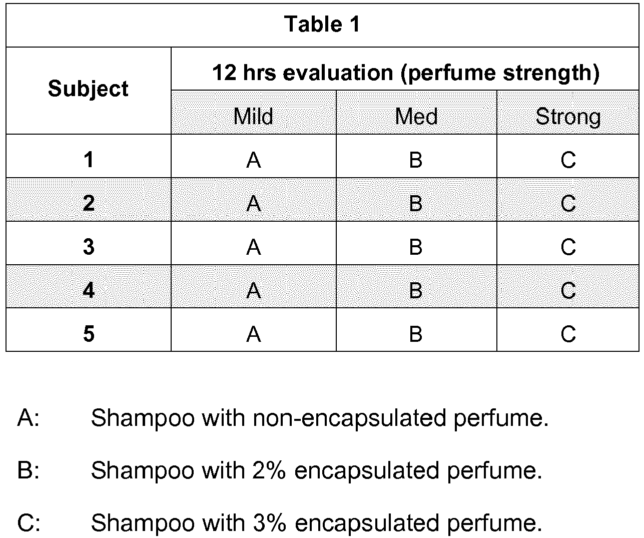

100% of the panelists ranked the strength of perfume of C as the strongest, followed by B then A as the mildest.

### PRODUCT EXAMPLES

### Fabric Softener Formulation

| Ingredient | Function | wt% |
|---|---|---|
| Praepagen TQ (Triethanolamine Esterquat)¹ | Cationic softener | 8 |
| Distilled water | Carrier | up to 100 |
| Colour [Sanolin Blue AE90]¹ | Color | 3 |
| Encapsulated fragrance according to the invention | | 0.3-10% |
| Perlogen 3000 (glycol distearate, laureth-4, cocamidopropyl betaine)¹ | Sheen & shine | 1 |
| Genapol LT (PEG-150 polyglyceryl-2 tristearate, laureth-3 and dipropylene glycol)¹ | Liquid thickener | 0.5 |

| | | |
|---|---|---|
| ¹Available from Clariant. | | |

### Hair Shampoo Formulation

| Ingredient | Function | wt% |
|---|---|---|
| Cocoamide DEA or MEA | Thickener | 5-10 |
| Alcohol ethoxy glyceryl sulfonate | Surfactant | 2-25 |
| Sodium or ammonium lauryl sulfate | Surfactant | 5-20 |
| Cocoamidopropyl betaine | Foaming agent | 0-20 |
| Polysorbates (Tween 20, 40, 60) | Solubilizer | 0-5 |
| Encapsulated fragrance according to the invention | | 0.3-10 |

## Claims

1. Core-shell microcapsule comprising a cross-linked polymeric shell encapsulating a perfume-containing core, wherein the cross-linked polymeric shell is the reaction product of a functionalized polysiloxane, a polyisocyanate and a polyamine and wherein the functionalized polysiloxane comprises a polysiloxane backbone, one or more ethoxylated alcohol side chains and one or more substituted or unsubstituted alkyl or aliphatic cycloalkyl side chains.

2. The core-shell microcapsule of claim 1, wherein the functionalized polysiloxane has Structure I: 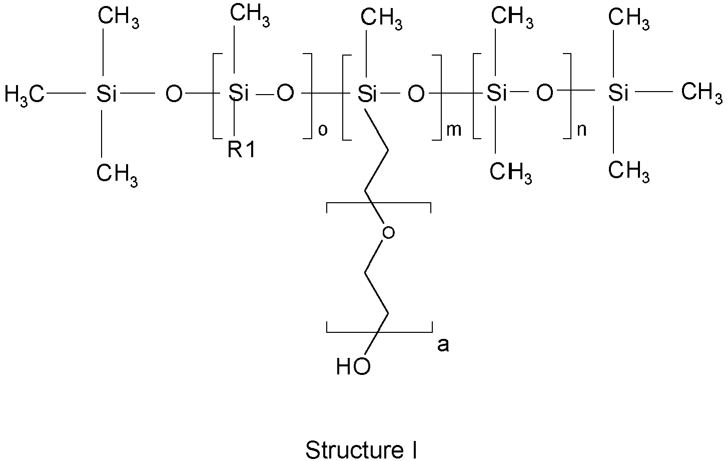 where:
R1 is a substituted or unsubstituted alkyl or is a substituted or unsubstituted aliphatic cycloalkyl group having from 1 to 20 carbon atoms;
a is from 1 to 25, preferably 5 to 15;
m+n+o is 100; m+o is from 10 to 30; and n is from 70 to 90.

3. The core-shell microcapsule of claim 2, wherein in Structure I:
R1 is a straight chain C16 alkyl group;
a is 10;
n is 75;
m+o is 25.

4. The core-shell microcapsule of any of claims 1 to 3, wherein the polyisocyanate is selected from an aromatic polyisocyanate, an aliphatic polyisocyanate and mixtures thereof.

5. The core-shell microcapsule of any preceding claim, wherein the polyisocyanate is selected from isophorone diisocyanate, toluene diisocyanate, hexamethylene diisocyanate, toluene triisocyanate, 1,4-phenylene diisocyanate, 1,3-phenylene diisocyanate, m-xylylene diisocyanate, tolylene-2,4-diisocyanate, tolylene-2,6-diisocyanate, poly(hexamethylene diisocyanate), trans-1,4-cyclohexylene diisocyanate, polypropylene glycol) terminated with tolylene 2,4-diisocyanate, tolylene diisocyanate, 1,4-diisocyanatobutane, 2,4-toluene diisocyanate, 2,6-toluene diisocyanate, 1,8-diisocyanatooctane, 3,3'-dimethyl-4,4'-biphenylene diisocyanate, 4,4'-Methylenebis(phenyl isocyanate) and mixtures thereof.

6. The core-shell microcapsule of any preceding claim, wherein the polyisocyanate comprises isophorone diisocyanate.

7. The core-shell microcapsule of any preceding claim, wherein the polyamine is selected from diethylenetriamine, bis(hexamethylene)triamine, triamine tetraacetate, melamine-(triamine-15N3), bis(hexamethylene)triamine, (3-trimethoxy silyl propyl) diethylenetriamine, N-cyclopropyl-2,4,6-triamino-1,3,5-triazine, melamine-13C3, N(2),N(4),N(6)-tris(2-phenylethyl)-1,3,5-triazine-2,4,6-triamine, N(2),N(4),N(6)-tris(4-chlorophenyl)-1,3,5-triazine-2,4,6-triamine, N,N'-di-tert-butyl-N", N,N-dimethyl-1,3,5-triazine-2,4,6-triamine and mixtures thereof.

8. The core-shell microcapsule of any preceding claim, wherein the polyamine comprises diethylenetriamine.

9. The core-shell microcapsule of any preceding claim having a diameter of 0.1 to 100 micrometers, preferably from 1 to 30 micrometers.

10. An aqueous dispersion or emulsion comprising core-shell microcapsules of any preceding claim.

11. A personal care or fabric care product comprising core-shell microcapsules of any of claims 1 to 9.

12. A method of making an aqueous dispersion of core-shell microcapsules comprising a cross-linked polymeric shell encapsulating a perfume-containing core, the method comprising:
a. Forming a disperse phase comprising a perfume and a polyisocyanate;
b. Forming an aqueous continuous phase;
c. Combining the disperse phase with the aqueous continuous phase and mixing to form a emulsion of the dispersed phase in the aqueous continuous phase;
d. Adding a functionalized polysiloxane to the emulsion and mixing, wherein the functionalized polysiloxane comprises a polysiloxane backbone, at least one ethoxylated alcohol side chain and one or more substituted or unsubstituted alkyl or aliphatic cycloalkyl side chains;
e. Adding a polyamine to the mixture and mixing to form core-shell microcapsules dispersed in the aqueous continuous phase.

13. The method of claim 12 comprising adding a material selected from an emulsifier, a catalyst, a rheology modifier, and mixtures thereof in b. or c.

14. The method of claim 12 or 13, wherein the functionalized polysiloxane has Structure I: 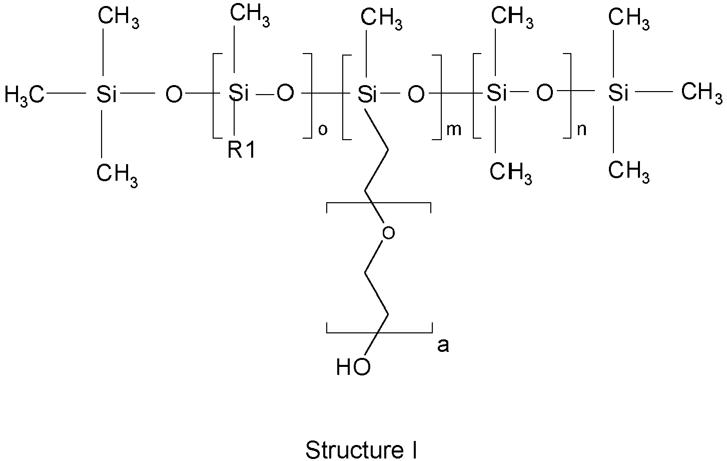 where:
R1 is a substituted or unsubstituted alkyl or is a substituted or unsubstituted aliphatic cycloalkyl group having from 1 to 20 carbon atoms;
a is from 1 to 25, preferably 5 to 15;
m+n+o is 100; m+o is from 10 to 30; and n is from 70 to 90.

15. The method of claim 14, wherein in Structure I:
R1 is a straight chain C16 alkyl group;
a is 10;
n is 75;
m+o is 25.

16. The method of any of claims 12 to 15, wherein the polyisocyanate is selected from isophorone diisocyanate, toluene diisocyanate, hexamethylene diisocyanate, toluene triisocyanate, 1,4-phenylene diisocyanate, 1,3-phenylene diisocyanate, m-xylylene diisocyanate, tolylene-2,4-diisocyanate, tolylene-2,6-diisocyanate, poly(hexamethylene diisocyanate), trans-1,4-cyclohexylene diisocyanate, poly(propylene glycol) terminated with tolylene 2,4-diisocyanate, tolylene diisocyanate, 1,4-diisocyanatobutane, 2,4-toluene diisocyanate, 2,6-toluene diisocyanate, 1,8-diisocyanatooctane, 3,3'-dimethyl-4,4'-biphenylene diisocyanate, 4,4'-Methylenebis(phenyl isocyanate) and mixtures thereof.

17. The method of any of claims 12 to 16, wherein the polyisocyanate comprises isophorone diisocyanate.

18. The method of any of claims 12 to 17, wherein the polyamine is selected from diethylenetriamine, bis(hexamethylene)triamine, triamine tetraacetate, melamine-(triamine-15N3), bis(hexamethylene)triamine, (3-trimethoxy silyl propyl) diethylenetriamine, N-cyclopropyl-2,4,6-triamino-1,3,5-triazine, melamine-13C3, N(2),N(4),N(6)-tris(2-phenylethyl)-1,3,5-triazine-2,4,6-triamine, N(2),N(4),N(6)-tris(4-chlorophenyl)-1,3,5-triazine-2,4,6-triamine, N,N'-di-tert-butyl-N", N,N-dimethyl-1,3,5-triazine-2,4,6-triamine and mixtures thereof.

19. The method of any of claims 12 to 18, wherein the polyamine comprises diethylenetriamine.

## Patentansprüche

1. Kern-Schale-Mikrokapsel, die eine einen parfümhaltigen Kern verkapselnde vernetzte Polymerschale umfasst, wobei es sich bei der vernetzten Polymerschale um das Reaktionsprodukt eines funktionalisierten Polysiloxans, eines Polyisocyanats und eines Polyamins handelt und wobei das funktionalisierte Polysiloxan ein Polysiloxangrundgerüst, eine oder mehrere Seitenketten von ethoxyliertem Alkohol und eine oder mehrere Seitenketten von substituiertem oder unsubstituiertem Alkyl oder aliphatischem Cycloalkyl umfasst.

2. Kern-Schale-Mikrokapsel nach Anspruch 1, wobei das funktionalisierte Polysiloxan die Struktur I aufweist: 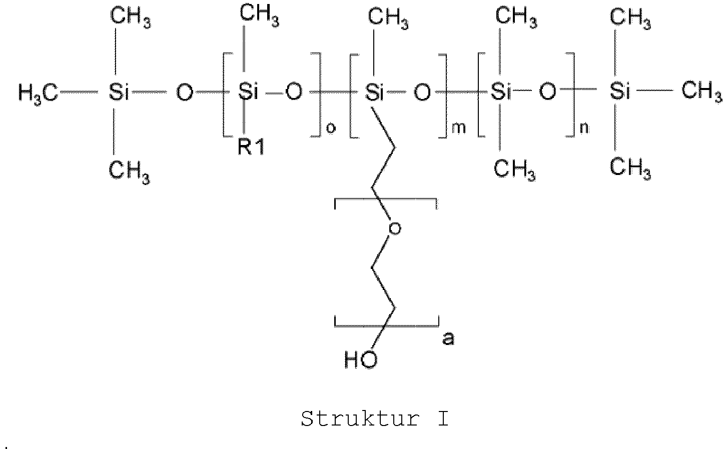 worin:
R1 für ein substituiertes oder unsubstituiertes Alkyl steht oder für eine substituierte oder unsubstituierte aliphatische Cycloalkylgruppe mit 1 bis 20 Kohlenstoffatomen steht;
a 1 bis 25, vorzugsweise 5 bis 15, ist;
m+n+o 100 ist; m+o 10 bis 30 ist; und n 70 bis 90 ist.

3. Kern-Schale-Mikrokapsel nach Anspruch 2, wobei in Struktur I:
R1 für eine geradkettige C16-Alkylgruppe steht;
a 10 ist;
n 75 ist;
m+o 25 ist.

4. Kern-Schale-Mikrokapsel nach einem der Ansprüche 1 bis 3, wobei das Polyisocyanat aus einem aromatischen Polyisocyanat, einem aliphatischen Polyisocyanat und Mischungen davon ausgewählt ist.

5. Kern-Schale-Mikrokapsel nach einem der vorhergehenden Ansprüche, wobei das Polyisocyanat aus Isophorondiisocyanat, Toluoldiisocyanat, Hexamethylendiisocyanat, Toluoltriisocyanat, 1,4-Phenylendiisocyanat, 1,3-Phenylendiisocyanat, m-Xylylendiisocyanat, Tolylol-2,4-diisocyanat, Tolylol-2,6-diisocyanat, Poly(hexamethylendiisocyanat), trans-1,4-Cyclohexylendiisocyanat, mit Toluylen-2,4-diisocyanat terminiertem Poly(propylenglykol), Toluylendiisocyanat, 1,4-Diisocyanatobutan, 2,4-Toluoldiisocyanat, 2,6-Toluoldiisocyanat, 1,8-Diisocyanatooctan, 3,3'-Dimethyl-4,4'-biphenylendiisocyanat, 4,4'-Methylenbis(phenylisocyanat) und Mischungen davon ausgewählt ist.

6. Kern-Schale-Mikrokapsel nach einem der vorhergehenden Ansprüche, wobei das Polyisocyanat Isophorondiisocyanat umfasst.

7. Kern-Schale-Mikrokapsel nach einem der vorhergehenden Ansprüche, wobei das Polyamin aus Diethylentriamin, Bis(hexamethylen)triamin, Triamintetraacetat, Melamin-(triamin-15N3), Bis(hexamethylen)triamin, (3-Trimethoxysilylpropyl)diethylentriamin, N-Cyclopropyl-2,4,6-triamino-1,3,5-triazin, Melamin-13C3, N(2),N(4),N(6)-Tris(-2-phenylethyl)-1,3,5-triazin-2,4,6-triamin, N(2),N(4),N(6)-Tris(-4-chlorphenyl)-1,3,5-triazin-2,4,6-triamin, N,N'-Di-tert-butyl-N'', N,N-Dimethyl-1,3,5-triazin-2,4,6-triamin und Mischungen davon ausgewählt ist.

8. Kern-Schale-Mikrokapsel nach einem der vorhergehenden Ansprüche, wobei das Polyamin Diethylentriamin umfasst.

9. Kern-Schale-Mikrokapsel nach einem der vorhergehenden Ansprüche mit einem Durchmesser von 0,1 bis 100 Mikrometern, vorzugsweise 1 bis 30 Mikrometern.

10. Wässrige Dispersion oder Emulsion, umfassend Kern-Schale-Mikrokapseln nach einem der vorhergehenden Ansprüche.

11. Körperpflege- oder Textilpflegeprodukt, umfassend Kern-Schale-Mikrokapseln nach einem der Ansprüche 1 bis 9.

12. Verfahren zur Herstellung einer wässrigen Dispersion von Kern-Schale-Mikrokapseln, die eine einen parfümhaltigen Kern verkapselnde vernetzte Polymerschale umfasst, wobei das Verfahren Folgendes umfasst:
a. Bilden einer dispersen Phase, die ein Parfüm und ein Polyisocyanat umfasst;
b. Bilden einer wässrigen kontinuierlichen Phase;
c. Kombinieren der dispersen Phase mit der wässrigen kontinuierlichen Phase und Mischen zur Bildung einer Emulsion der dispergierten Phase in der wässrigen kontinuierlichen Phase;
d. Zugeben eines funktionalisierten Polysiloxans zu der Emulsion und Mischen, wobei das funktionalisierte Polysiloxan ein Polysiloxangrundgerüst, mindestens eine Seitenkette von ethoxyliertem Alkohol und eine oder mehrere Seitenketten von substituiertem oder unsubstituiertem Alkyl oder aliphatischem Cycloalkyl umfasst;
e. Zugeben eines Polyamins zu der Mischung und Mischen zur Bildung von in der wässrigen kontinuierlichen Phase dispergierten Kern-Schale-Mikrokapseln.

13. Verfahren nach Anspruch 12, umfassend das Zugeben eines aus einem Emulgator, einem Katalysator, einem Rheologiemodifikator und Mischungen davon ausgewählten Stoffs in b. oder c.

14. Verfahren nach Anspruch 12 oder 13, wobei das funktionalisierte Polysiloxan die Struktur I aufweist: 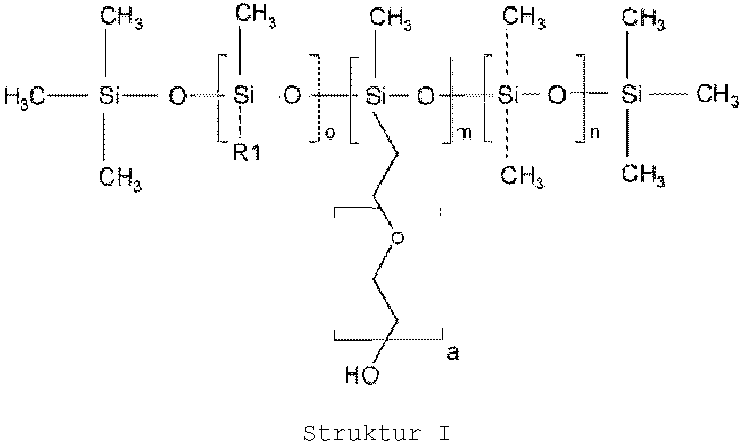 worin:
R1 für ein substituiertes oder unsubstituiertes Alkyl steht oder für eine substituierte oder unsubstituierte aliphatische Cycloalkylgruppe mit 1 bis 20 Kohlenstoffatomen steht;
a 1 bis 25, vorzugsweise 5 bis 15, ist;
m+n+o 100 ist; m+o 10 bis 30 ist; und n 70 bis 90 ist.

15. Verfahren nach Anspruch 14, wobei in Struktur I:
R1 für eine geradkettige C16-Alkylgruppe steht;
a 10 ist;
n 75 ist;
m+o 25 ist.

16. Verfahren nach einem der Ansprüche 12 bis 15, wobei das Polyisocyanat aus Isophorondiisocyanat, Toluoldiisocyanat, Hexamethylendiisocyanat, Toluoltriisocyanat, 1,4-Phenylendiisocyanat, 1,3-Phenylendiisocyanat, m-Xylylendiisocyanat, Tolylol-2,4-diisocyanat, Tolylol-2,6-diisocyanat, Poly(hexamethylendiisocyanat), trans-1,4-Cyclohexylendiisocyanat, mit Toluylen-2,4-diisocyanat terminiertem Poly(propylenglykol), Toluylendiisocyanat, 1,4-Diisocyanatobutan, 2,4-Toluoldiisocyanat, 2,6-Toluoldiisocyanat, 1,8-Diisocyanatooctan, 3,3'-Dimethyl-4,4'-biphenylendiisocyanat, 4,4'-Methylenbis(phenylisocyanat) und Mischungen davon ausgewählt ist.

17. Verfahren nach einem der Ansprüche 12 bis 16, wobei das Polyisocyanat Isophorondiisocyanat umfasst.

18. Verfahren nach einem der Ansprüche 12 bis 17, wobei das Polyamin aus Diethylentriamin, Bis(hexamethylen)triamin, Triamintetraacetat, Melamin-(triamin-15N3), Bis(hexamethylen)triamin, (3-Trimethoxysilylpropyl)diethylentriamin, N-Cyclopropyl-2,4,6-triamino-1,3,5-triazin, Melamin-13C3, N(2),N(4),N(6)-Tris(-2-phenylethyl)-1,3,5-triazin-2,4,6-triamin, N(2),N(4),N(6)-Tris(-4-chlorphenyl)-1,3,5-triazin-2,4,6-triamin, N,N'-Di-tert-butyl-N'', N,N-Dimethyl-1,3,5-triazin-2,4,6-triamin und Mischungen davon ausgewählt ist.

19. Verfahren nach einem der Ansprüche 12 bis 18, wobei das Polyamin Diethylentriamin umfasst.

## Revendications

1. Microcapsule de type noyau-enveloppe comprenant une enveloppe polymérique réticulée encapsulant un noyau contenant un parfum, l'enveloppe polymérique réticulée étant le produit de réaction d'un polysiloxane fonctionnalisé, d'un polyisocyanate et d'une polyamine et le polysiloxane fonctionnalisé comprenant un squelette de polysiloxane, une ou plusieurs chaînes latérales de type alcool éthoxylé et une ou plusieurs chaînes latérales de type alkyle ou cycloalkyle aliphatique, substituées ou non substituées.

2. Microcapsule de type noyau-enveloppe selon la revendication 1, le polysiloxane fonctionnalisé possédant la Structure I : 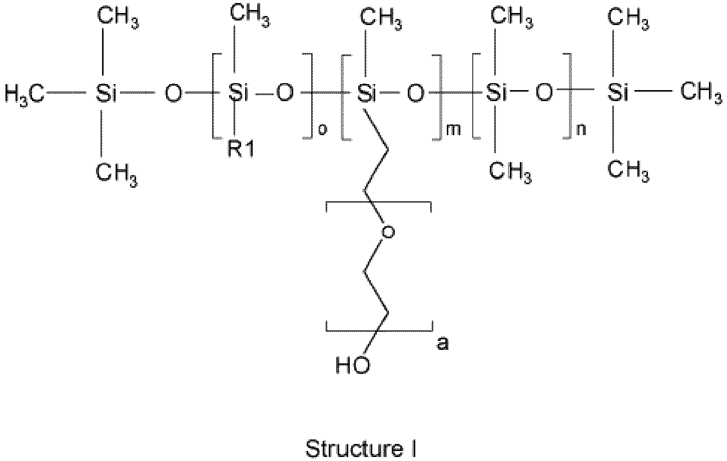 dans laquelle :
R1 est un alkyle substitué ou non substitué ou est un groupe cycloalkyle aliphatique substitué ou non substitué possédant 1 à 20 atomes de carbone ;
a est de 1 à 25, préférablement 5 à 15 ;
m + n + o est 100 ; m + o est de 10 à 30 ; et n est de 70 à 90.

3. Microcapsule de type noyau-enveloppe selon la revendication 2, dans laquelle dans la Structure I :
R1 est un groupe alkyle en C16 à chaîne droite ;
a est 10 ;
n est 75 ;
m + o est 25.

4. Microcapsule de type noyau-enveloppe selon l'une quelconque des revendications 1 à 3, le polyisocyanate étant choisi parmi un polyisocyanate aromatiques, un polyisocyanate aliphatique et des mélanges correspondants.

5. Microcapsule de type noyau-enveloppe selon une quelconque revendication précédente, le polyisocyanate étant choisi parmi un diisocyanate d'isophorone, un diisocyanate de toluène, le diisocyanate d'hexaméthylène, un triisocyanate de toluène, le diisocyanate de 1,4-phénylène, le diisocyanate de 1,3-phénylène, le diisocyanate de m-xylylène, le 2,4-diisocyanate de tolylène, le 2,6-diisocyanate de tolylène, un poly(diisocyanate d'hexaméthylène), le diisocyanate de trans-1,4-cyclohexylène, un poly(propylène glycol) terminé par 2,4-diisocyanate de tolylène, un diisocyanate de tolylène, le 1,4-diisocyanatobutane, le 2,4-diisocyanate de toluène, le 2,6-diisocyanate de toluène, le 1,8-diisocyanatooctane, le 4,4'-diisocyanate de 3,3'-diméthyl-biphénylène, le 4,4'-méthylènebis(phényle isocyanate) et des mélanges correspondants.

6. Microcapsule de type noyau-enveloppe selon une quelconque revendication précédente, le polyisocyanate comprenant un diisocyanate d'isophorone.

7. Microcapsule de type noyau-enveloppe selon une quelconque revendication précédente, la polyamine étant choisie parmi la diéthylènetriamine, la bis(hexaméthylène)triamine, un tétraacétate de triamine, la mélamine-(triamine-15N3), la bis(hexaméthylène)triamine, la (3-triméthoxysilylpropyl)diéthylènetriamine, la N-cyclopropyl-2,4,6-triamino-1,3,5-triazine, la mélamine-13C3, la N(2),N(4),N(6)-tris(2-phényléthyl)-1,3,5-triazine-2,4,6-triamine, la N(2),N(4),N(6)-tris(4-chlorophényl)-1,3,5-triazine-2,4,6-triamine, la N,N'-di-tert-butyl-N",N,N-diméthyl-1,3,5-triazine-2,4,6-triamine et des mélanges correspondants.

8. Microcapsule de type noyau-enveloppe selon une quelconque revendication précédente, la polyamine comprenant de la diéthylènetriamine.

9. Microcapsule de type noyau-enveloppe selon une quelconque revendication précédente possédant un diamètre de 0,1 à 100 micromètres, préférablement de 1 à 30 micromètres.

10. Dispersion ou émulsion aqueuse comprenant des microcapsules de type noyau-enveloppe selon une quelconque revendication précédente.

11. Produit de soin personnel ou d'entretien de tissus comprenant des microcapsules de type noyau-enveloppe selon l'une quelconque des revendications 1 à 9.

12. Procédé de préparation d'une dispersion aqueuse de microcapsules de type noyau-enveloppe comprenant une enveloppe polymérique réticulée encapsulant un noyau contenant un parfum, le procédé comprenant :
a. la formation d'une phase dispersée comprenant un parfum et un polyisocyanate ;
b. la formation d'une phase continue aqueuse ;
c. la combinaison de la phase dispersée avec la phase continue aqueuse et le mélange pour former une émulsion de la phase dispersée dans la phase continue aqueuse ;
d. l'ajout d'un polysiloxane fonctionnalisé à l'émulsion et le mélange, le polysiloxane fonctionnalisé comprenant un squelette de polysiloxane, au moins une chaîne latérale de type alcool éthoxylé et une ou plusieurs chaînes latérales de type alkyle ou cycloalkyle aliphatique, substituées ou non substituées ;
e. l'ajout d'une polyamine au mélange et le mélange pour former des microcapsules de type noyau-enveloppe dispersées dans la phase continue aqueuse.

13. Procédé selon la revendication 12 comprenant l'ajout d'un matériau choisi parmi un émulsifiant, un catalyseur, un modificateur de rhéologie, et des mélanges de ceux-ci en b. ou c..

14. Procédé selon la revendication 12 ou 13, le polysiloxane fonctionnalisé possédant la Structure I : 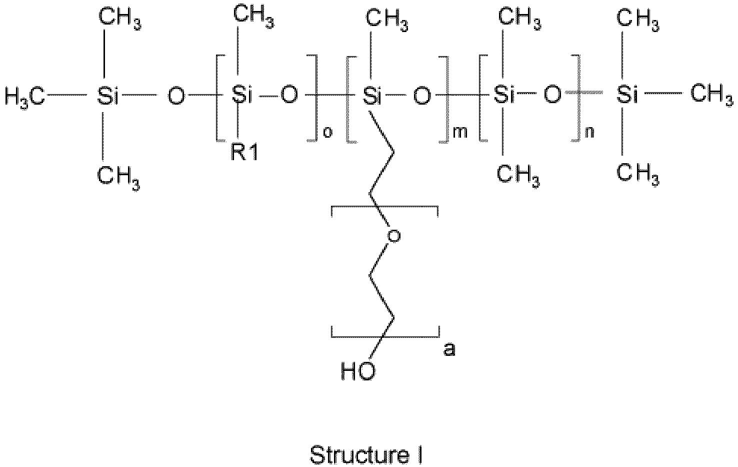 dans laquelle :
R1 est un alkyle substitué ou non substitué ou est un groupe cycloalkyle aliphatique substitué ou non substitué possédant 1 à 20 atomes de carbone ;
a est de 1 à 25, préférablement 5 à 15 ;
m + n + o est 100 ; m + o est de 10 à 30 ; et n est de 70 à 90.

15. Procédé selon la revendication 14, dans lequel dans la Structure I :
R1 est un groupe alkyle en C16 à chaîne droite ;
a est 10 ;
n est 75 ;
m + o est 25.

16. Procédé selon l'une quelconque des revendications 12 à 15, le polyisocyanate étant choisi parmi un diisocyanate d'isophorone, un diisocyanate de toluène, le diisocyanate d'hexaméthylène, un triisocyanate de toluène, le diisocyanate de 1,4-phénylène, le diisocyanate de 1,3-phénylène, le diisocyanate de m-xylylène, le 2,4-diisocyanate de tolylène, le 2,6-diisocyanate de tolylène, un poly(diisocyanate d'hexaméthylène), le diisocyanate de trans-1,4-cyclohexylène, un poly(propylène glycol) terminé par 2,4-diisocyanate de tolylène, un diisocyanate de tolylène, le 1,4-diisocyanatobutane, le 2,4-diisocyanate de toluène, le 2,6-diisocyanate de toluène, le 1,8-diisocyanatooctane, le 4,4'-diisocyanate de 3,3'-diméthyl-biphénylène, le 4,4'-méthylènebis(phényle isocyanate) et des mélanges correspondants.

17. Procédé selon l'une quelconque des revendications 12 à 16, le polyisocyanate comprenant un diisocyanate d'isophorone.

18. Procédé selon l'une quelconque des revendications 12 à 17, la polyamine étant choisie parmi la diéthylènetriamine, la bis(hexaméthylène)triamine, un tétraacétate de triamine, la mélamine-(triamine-15N3), la bis(hexaméthylène)triamine, la (3-triméthoxysilylpropyl)diéthylènetriamine, la N-cyclopropyl-2,4,6-triamino-1,3,5-triazine, la mélamine-13C3, la N(2),N(4),N(6)-tris(2-phényléthyl)-1,3,5-triazine-2,4,6-triamine, la N(2),N(4),N(6)-tris(4-chlorophényl)-1,3,5-triazine-2,4,6-triamine, la N,N'-di-tert-butyl-N",N,N-diméthyl-1,3,5-triazine-2,4,6-triamine et des mélanges correspondants.

19. Procédé selon l'une quelconque des revendications 12 à 18, la polyamine comprenant de la diéthylènetriamine.
